# EUROPÄISCHE PATENTSCHRIFT

(11) **EP 0 696 330 B1**
(45) Veröffentlichungstag und Bekanntmachung des Hinweises auf die Patenterteilung: **12.11.1997**
(21) Anmeldenummer: 95906835.4
(22) Anmeldetag: 01.02.1995
(51) Int. Cl.: D01F 13/02, D01F 2/00, B01J 39/22, B01J 41/16, C08B 1/00

(54) **VERFAHREN ZUR REINIGUNG WÄSSRIGER LÖSUNGEN TERTIÄRER AMINOXIDE**
PROCESS FOR PURIFYING AQUEOUS SOLUTIONS OF TERTIARY AMINO OXIDES
PROCEDE DE PURIFICATION DE SOLUTIONS AQUEUSES D'AMINO-OXYDES TERTIAIRES

(30) Priorität: 01.03.1994 AT 430/94
(43) Veröffentlichungstag der Anmeldung: 14.02.1996
(73) Patentinhaber: LENZING AKTIENGESELLSCHAFT, 4860 Lenzing (AT)
(72) Erfinder: MÜLLEDER, Eduard, A-4030 Linz (AT); FIRGO, Heinrich, A-4840 Vöcklabruck (AT)
(74) Vertreter: Schwarz, Albin, Dr. Kopecky & Schwarz Patentanwälte
(86) Internationale Anmeldenummer: AT9500022
(87) Internationale Veröffentlichungsnummer: WO9523885

(56) Entgegenhaltungen:
- EP-A- 0 427 701
- WO-A-89/09643
- WO-A-93/11287

## Beschreibung

Die Erfindung betrifft ein Verfahren zur Reinigung wäßriger Lösungen tertiärer Aminoxide.

Seit einigen Jahrzehnten wird nach Verfahren zur Herstellung cellulosischer Formkörper gesucht, welche das heute in großem Maßstab angewendete Viskoseverfahren ersetzen sollen. Als eine nicht zuletzt wegen einer besseren Umweltverträglichkeit interessante Alternative hat sich dabei herauskristallisiert, Cellulose ohne Derivatisierung in einem organischen Lösungsmittel aufzulösen und aus dieser Lösung Formkörper, z.B. Fasern und Folien, zu extrudieren. Solcherart extrudierte Fasern erhielten von der BISFA (The International Bureau for the Standardization of man made fibers) den Gattungsnamen Lyocell. Unter einem organischen Lösungsmittel wird von der BISFA ein Gemisch aus einer organischen Chemikalie und Wasser verstanden.

Es hat sich herausgestellt, daß sich als organisches Lösungsmittel insbesondere ein Gemisch aus einem tertiären Aminoxid und Wasser sehr gut zur Herstellung von cellulosischen Formkörpern eignet. Als Aminoxid wird dabei in erster Linie N-Methylmorpholin-N-oxid (NMMO) verwendet. Andere Aminoxide sind z.B. in der EP-A - 0 533 070 beschrieben. Ein Verfahren zur Herstellung formbarer Celluloselösungen ist z.B. aus der EP-A - 0 365 419 bekannt.

Die Cellulose wird aus der geformten Celluloselösung in einem wäßrigen Fällbad gefällt. Dabei reichert sich das Fällbad mit Aminoxid an. Für die Wirtschaftlichkeit des Verfahrens ist es von entscheidender Bedeutung, daß das Aminoxid nahezu vollständig zurückgewonnen und wiederverwendet wird. Das Aminoxidverfahren weist somit die folgenden 3 Haupt schritte auf:
(A) Auflösen von Cellulose in einer wäßrigen Lösung eines tertiären Aminoxids, insbesondere N-Methylmorpholin-N-oxid (NMMO), um eine formbare Celluloselösung zu bilden,
(B) Formen der Celluloselösung und Führen der geformten Celluloselösung in ein wäßriges Fällbad, in welchem die Cellulose gefällt wird, wodurch ein Formkörper und ein gebrauchtes Fällbad gebildet werden,
(C) Regenerieren, d.h. Reinigen und Konzentrieren, des gebrauchten Fällbades, wobei eine regenierte, wäßrige Aminoxidlösung gebildet wird, die im Schritt (A) erneut zur Auflösung von Cellulose eingesetzt wird.

Im Fällbad reichert sich jedoch nicht nur Aminoxid sondern auch Abbauprodukte der Cellulose und des Aminoxids an. Diese können stark gefärbt sein und die Qualität der hergestellten Formkörper beeinträchtigen, wenn sie nicht aus dem Fällbad entfernt werden. Zusätzlich können sich im Fällbad auch Metallspuren anreichern, die zu einer Verminderung der Prozeßsicherheit führen.

Für das Entfernen dieser Abbauprodukte vor dem erneuten Einsetzen der Aminoxidlösung im Schritt (A) sind aus der Literatur einige Vorschläge bekannt:

Die DD-A 254 199 beschreibt ein Verfahren zur Reinigung wäßriger Lösungen von NMMO, gemäß welchem die Lösung Anionenaustauscher passiert, wobei in einer ersten Stufe der Anionenaustauscher ein mit tertiären Aminogruppen vom Typ -CH₂N(CH₃)₂ besetztes Austauscherharz eines Styrol/Divinylbenzol-Copolymerisates enthält und in einer zweiten Stufe als funktionelle Gruppen quaternäre Ammoniumgruppen vom Typ -CH₂N(CH₃)₃OH enthält. Es wird beschrieben, daß die zu reinigende NMMO-Lösung zu Beginn der Reinigung dunkel, nach der ersten Stufe braun bis gelb und nach der zweiten Stufe hellgelb bis wasserklar ist.

Ein Nachteil dieses Verfahrens besteht darin, daß die solcherart behandelten Lösungen einen hohen pH-Wert aufweisen, der in weiterer Folge zu einem erhöhten Reinigungsaufwand führt. Dazu kommt noch, daß bei diesem vorbekannten Verfahren Alkali- und Erdalkalikationen nicht aus der Lösung entfernt werden. Die Metallionen bzw. die Alkali- und Erdalkaliionen führen zu unerwünschten Ablagerungen und Verkrustungen, zu ungelösten störenden Stoffen in der Lösung sowie zu einer Verminderung der Prozeßsicherheit. Es ist zwar möglich, diese Salze durch Zugabe eines Fällungsmittels mit anschließender Filtration oder mit anderen Trennmitteln zu entfernen, diese Arbeitsgänge tragen jedoch wiederum zusätzliche Chemikalien ein bzw. bedeuten einen zusätzlichen technischen Aufwand.

In der EP-A - 0 427 701 ist ein Verfahren zur Reinigung von wäßrigen Aminoxidlösungen beschrieben, gemäß welchem die Reinigung in einem einstufigen Verfahren mit einem Anionenaustauscher durchgeführt wird, der als funktionelle Gruppen ausschließlich quarternäre Tetraalkylammoniumgruppen der Formeln -CH₂N⁺(CH₃)₃X⁻ oder -CH₂N⁺(CH₃)₂(CH₂OH)]X⁻ aufweist, wobei X⁻ das Anion einer anorganischen oder organischen Säure darstellt, worauf der Anionenaustauscher mit einer wäßrigen sauren Lösung regeneriert wird. Das Anion X⁻ stammt vorzugsweise von einer flüchtigen Säure, insbesondere Kohlensäure, Ameisensäure oder Essigsäure. Diese Säuren werden auch zur Regenerierung des Anionenaustauschers vorgeschlagen.

In der Internationalen Patentanmeldung WO93/11287 wird vorgeschlagen, die Regenerierung eines stark basischen Anionenaustauschers, der als Trägermaterial ein Styrol/Divinylbenzol-Copolymerisat aufweist, mit einer wäßrigen Lösung einer starken anorganischen Säure und anschließend mit Natronlauge, das heißt zweistufig, durchzuführen. Die Regenerierung muß zweistufig durchgeführt werden, weil der Anionenaustauscher von der zu reinigenden Lösung so stark verfärbt wird, daß eine bloße Regenerierung mit Natronlauge nicht ausreichend ist, um das Harz wieder zu entfärben und die Kapazität aufrecht zu erhalten. Dies gelingt nur, wenn es zusätzlich mit einer starken anorganischen Säure behandelt wird.

Die zweistufige Behandlung führt zu einem gesteigerten Chemikalieneinsatz sowie zum Verwenden stark reizender Substanzen, wie z.B. Salzsäure. Zusätzlich ist dem Beispiel 5 dieser Literatur zu entnehmen, daß selbst bei Anwendung dieses zweistufigen Regenerationsverfahrens die Kapazität des Anionenaustauschers nach 10 Cyclen auf die Hälfte des ursprünglichen Wertes gesunken ist.

Ein weiterer Nachteil der als Trägermaterial in ionenaustauschenden Systemen verbreitet eingesetzten Styrol/Divinylbenzol-Copolymerisate besteht darin, daß sie unverrottbar und einer Wiederverwertung nur schwer zugänglich sind, sodaß sie bei einem nach gewissen Zeitspannen erforderlichen Austausch des ionenaustauschenden Systems als Sondermüll verbrannt werden müssen.

Die in allgemeinen technischen Handbüchern (siehe z.B. Ullmann's "Encyclopedia of Industrial Chemistry", Band A 14, Seote 396) angegebenen alternativen Trägermaterialien, wie z.B. Polyacrylmaterialien, Phenol/Formaldehyd-Harze oder Polyalkylamin-Harze, weisen diesen Nachteil ebenfalls auf und zwingen zudem meistens, wie z.B. im Fall von Phenol/Formaldehyd-Harzen, zum Einsatz von giftigen oder die Umwelt belastenden Ausgangsmaterialien.

Die Aufgabe der vorliegenden Erfindung besteht nun darin, für das Aminoxidverfahren Ionenaustauscher vorzusehen, mit denen gebrauchte NMMO-Lösungen, wie zum Beispiel gebrauchte Fällbäder, gereinigt werden können und die die Nachteile von Styrol/Divinylbenzol-Copolymerisaten nicht aufweisen. Der zur Entfärbung verwendete Stoff soll somit einstufig vollkommen von den ihm anhaftenden gefärbten Substanzen befreit, also entfärbt werden können.

Das erfindungsgemäße Verfahren zur Herstellung cellulosischer Formkörper weist die folgenden Schritte auf:
(A) Auflösen von Cellulose in einer wäßrigen Lösung eines tertiären Aminoxids, insbesondere N-Methylmorpholin-N-oxid (NMMO), um eine formbare Celluloselösung zu bilden,
(B) Formen der Celluloselösung und Führen der geformten Celluloselösung in ein wäßriges Fällbad, in welchem die Cellulose gefällt wird, wodurch ein Formköper und ein gebrauchtes Fällbad gebildet werden,
(C) Reinigen des gebrauchten Fällbades, indem das Fällbad mit einem Ionenaustauscher in Kontakt gebracht wird, wobei eine gereinigte, wäßrige Aminoxidlösung gebildet wird, die, gegebenenfalls nach Konzentrierung, im Schritt (A) erneut zur Auflösung von Cellulose eingesetzt wird,
und ist dadurch gekennzeichnet,
daß ein Ionenaustauscher eingesetzt wird, dessen Träger Cellulose ist, an welche zum Ionenaustausch befähigte Gruppen angebracht sind.

Eine bevorzugte Ausgestaltung des erfindungsgemäßen Verfahrens besteht darin, daß am Träger Gruppen vorgesehen sind, die zum Anionenaustausch befähigt sind, und zwar insbesondere tertiäre Aminogruppen und/oder quaternäre Ammoniumgruppen.

Eine weitere bevorzugte Ausgestaltung des erfindungsgemäßen Verfahrens besteht darin, daß am Träger Gruppen vorgesehen sind, die zum Kationenaustausch befähigt sind, und zwar insbesondere Sulfonsäuregruppen und/oder Carboxylgruppen.

Die Erfindung betrifft ferner die Verwendung von Cellulose, welche mit Gruppen funktionalisiert ist, welche zum Ionenaustausch befähigt sind, zum Reinigen einer wäßrigen Lösung eines tertiären Aminoxides, insbesondere eines Fällbades, welches als tertiäres Aminoxid N-Methylmorpholin-N-oxid enthält.

Es hat sich überraschendenweise gezeigt, daß durch die Verwendung von an sich als Trägermaterial für Ionenaustauscher bekannten cellulosischen Materialien im Aminoxidverfahren ausgezeichnete Reinigungsergebnisse erzielt werden können, daß das eingesetzte Material einstufig regeneriert werden kann und daß zusätzlich der Vorteil gegeben ist, daß die Cellulose ein biologisch abbaubares Polymer ist.

Wie bereits erwähnt, ist Cellulose als Trägermaterial, welches ionenaustauschende Gruppen trägt, bekannt. In "Die chemische Behandlung und Modifizierung der Zellulose" (Rogowin und Galbraich; Georg Thieme Verlag, 1983) finden sich ab Seite 97 Hinweise auf den Einsatz von modifizierter Cellulose in Ionenaustauschern. Es werden mit Carboxy- und Sulfogruppen modifizierte Cellulosen zum Einsatz in Kationenaustauschern und mit Polyvinlypyridin modifizierte Cellulosen zum Einsatz in Anionenaustauschern beschrieben. Ferner wird der Einsatz dieser Anionenaustauscher als Filtermaterial für Universalgasmasken oder zum Entfärben von Agar-Agar-Lösungen in Verbindung mit Kationenaustauschern erwähnt.

Die gute Reinigungswirkung der cellulosichen Materialen ist im Aminoxidverfahren insofern überraschend, als in der zu reinigenden NMMO-Lösung eine Vielzahl unterschiedlicher chemischer Substanzen enhalten ist, deren chemisches Verhalten noch nicht in allen Einzelheiten geklärt ist. Ferner konnte nicht erwartet werden, daß der erfindungsgemäß verwendete Ionenaustauscher, im Gegensatz zu den für das Aminoxidverfahren im Stand der Technik vorgeschlagenen Styrol/Divinylbenzol-Copolymerisaten, nach Gebrauch auf einstufige Weise wieder vollkommen regeneriert werden kann, und daß somit keine irreversiblen Verfärbungen auftreten. Auf die z.B. aus der WO93/11287 bekannte zweite Reinigungsstufe mit einer starken Säure kann somit gänzlich verzichtet werden. Selbst eine Reinigung mit anderen, z.B. flüchtigen organischen Säuren ist nicht erforderlich.

Es wurde ferner gefunden, daß die Aufnahmekapazität der erfindungsgemäß verwendeten cellulosischen Materialien derjenigen herkömmlicher Materialien, z.B. eines Styrol/Divinylbenzol-Copolymerisates, entspricht.

Die Erfindung wird mit den folgenden Beispielen noch näher erläutert,
(A) Herstellung eines cellulosischen Materials mit quaternären Ammoniumgruppen als funktionelle Gruppen
Cellulose (27 g, "Viscokraft", Siebfraktion < 100µm), (3-Chlor-2-hydroxy)-N,N,N-trimethylpropanammoniumchlorid (55,1 g, DEGUSSA, Aktivgehalt 57%), NaOH (13,36 g) und 150 ml Wasser wurden gemischt und 24 Stunden gerührt. Anschließend wurde mit 1% HCl neutralisiert, filtriert, der Rückstand mit Wasser gewaschen und im Trockenschrank bei 50°C getrocknet.
(B) Bestimmung der Kapazität der modifizierten Cellulose beim Einsatz als Anionenaustauscher
5 g der in (A) hergestellten modifizierten Cellulose wurden mit einer wäßrigen, salzhaltigen Lösung, welche 20% NMMO enthielt, beladen. Es wurden Fraktionen von jeweils 10 ml aufgefangen und die Ionenkonzentration bestimmt. Die Differenz der absoluten Ionenmengen aus dem Zulauf pro Fraktion und dem Eluat pro Fraktion ergibt die an der modifizierten Cellulose adsorbierten Menge. Sämtliche Werte in den Tabellen 1 und 2 sind mg/l, mit Ausnahme der "Fraktionen kumuliert", welche in ml angegeben sind.
Die zur Beladung verwendete NMMNO-Lösung wies den folgenden Salzgehalt auf:

**Tabelle 1**

| Formiat | Cl⁻ | NO₂ ⁻ | NO₃ ⁻ | SO₄ ²⁻ | Oxalat |
|---|---|---|---|---|---|
| 80 | 131 | 29 | 31 | 8100 | 59 |

In den einzelnen Fraktionen des Eluates wurden folgende Ionenkonzentrationen bestimmt:

**Tabelle 2**

| Fraktionen kumuliert | Formiat | Cl⁻ | NO₂ ⁻ | NO₃ ⁻ | SO₄ ²⁻ | Oxalat |
|---|---|---|---|---|---|---|
| 10 | 0,9 | 0,6 | 0,5 | 0,5 | 3 | 0 |
| 20 | 2,7 | 3,5 | 0,5 | 1 | 168 | 1 |
| 30 | 16 | 18 | 2 | 5 | 1180 | 8 |
| 40 | 70 | 86 | 8 | 27 | 5725 | 38 |
| 50 | 87 | 112 | 11 | 41 | 7422 | 50 |
| 60 | 89 | 116 | 12 | 44 | 7852 | 54 |
| 70 | 89 | 120 | 12 | 47 | 8120 | 57 |
| 80 | 91 | 121 | 12 | 48 | 8415 | 59 |
| 90 | 90 | 122 | 13 | 49 | 8604 | 62 |
| 100 | 93 | 124 | 12 | 49 | 8681 | 63 |

Aus der Summe der abgetrennten Bestandteile errechnet sich die Kapazität der modifizierten Cellulose als Ionenaustauscher mit 1,088 Milliäquivalent/g Cellulose.
(C) Entfärbung
5 g der in (A) hergestellten modifizierten Cellulose wurden in Wasser aufgeschlämmt, in eine chromatographische Säule gefüllt und mit 20 ml 0,5% NaOH in die OH -Form übergeführt.

Anschließend wurden der Säule portionsweise insgesamt 100 ml verbrauchte, gefärbte (Extinktion bei 470 nm: 2,52) wäßrige NMMO-Lösung, welche als Fällbad bei der Herstellung cellulosischer Fasern nach dem Aminoxidverfahren anfällt, aufgegeben und die Extinktion des Eluates in Fraktionen zu je 10 ml bei einer Wellenlänge von 470 nm gemessen. Die Ergebnisse sind in der Tabelle 3 angegeben.

**Tabelle 3**

| (Beladung) | | | |
|---|---|---|---|
| Eluat (ml; kumuliert) | Extinktion des Eluates | Eluat (ml; kumuliert) | Extinktion des Eluates |
| 10 | 0,00246 | 80 | 0,2140 |
| 20 | 0,0050 | 90 | 0,3016 |
| 30 | 0,0219 | 100 | 0,5173 |
| 40 | 0,0543 | | |
| 50 | 0,0897 | | |
| 60 | 0,1203 | | |
| 70 | 0,1953 | | |

Der Tabelle 3 ist zu entnehmen, daß erst nach Zugabe von insgesamt 90 ml gefärbter NMMO-Lösung ein merklicher Anstieg der Extinktion festzustellen ist. Die modifizierte Cellulose ist somit in ausgezeichneter Weise geeignet, die NMMO-Lösung zu entfärben.

Nach Zugabe der NMMO-Lösung wurde die beladene, modifizierte Cellulose mit Natronlauge regeneriert und wiederum die Extinktion des Eluates gemessen, wobei für die erste Messung 30 ml Eluat gesammelt wurden, für die zweite Messung 20 ml und für die dritte und vierte je weitere 30 ml Eluat gesammelt wurden. Die Ergebnisse für die Regenerierung sind in der Tabellen 4 angegeben.

**Tabelle 4**

| (Regenerierung) | |
|---|---|
| Eluat (ml; kumuliert) | Extinktion des Eluates |
| 30 ml | 0,7828 |
| 50 ml | 13,5 |
| 80 ml | 0,4215 |
| 110 ml | 0,01153 |

In Tabelle 4 ist ersichtlich, daß bei der Regenerierung mit NaOH nach insgesamt 110 ml Eluat praktisch keine Verfärbung des Eluates mehr gegeben ist. Ferner wies auch das Material des Ionenaustauschers, also die modifizierte Cellulose, keinerlei Verfärbungen mehr auf. Es ist somit im Gegensatz zu den aus dem Stand der Technik bekannten Verfahren möglich, den Ionenaustauscher, das heißt die modifizierte Cellulose, lediglich durch Regenerierung mit NaOH von den begleitenden Farbstoffen zu befreien und sie für einen nächsten Reinigungscyclus einsatzbereit zu machen.

Es hat sich gezeigt, daß die Regenerierung statt mit Natronlauge auch mit Alkoholen, z.B. Ethanol, vorgenommen werden kann.

## Patentansprüche

1. Verfahren zur Herstellung cellulosischer Formkörper, welches Verfahren folgende Schritte aufweist:
(A) Auflösen von Cellulose in einer wäßrigen Lösung eines tertiären Aminoxids, insbesondere N-Methylmorpholin-N-oxid, um eine formbare Celluloselösung zu bilden,
(B) Formen der Celluloselösung und Führen der geformten Celluloselösung in ein wäßriges Fällbad, in welchem die Cellulose gefällt wird, wodurch ein Formköper und ein gebrauchtes Fällbad gebildet werden,
(C) Reinigen des gebrauchten Fällbades, indem das Fällbad mit einem Ionenaustauscher in Kontakt gebracht wird, wobei eine gereinigte, wäßrige Aminoxidlösung gebildet wird, die, gegebenenfalls nach Konzentrierung, im Schritt (A) erneut zur Auflösung von Cellulose eingesetzt wird,
dadurch gekennzeichnet,
daß ein Ionenaustauscher eingesetzt wird, dessen Träger Cellulose ist, an welche zum Ionenaustausch befähigte Gruppen angebracht sind.

2. Verfahren nach Anspruch 1, dadurch gekennzeichnet, daß Gruppen vorgesehen sind, die zum Anionenaustausch befähigt sind.

3. Verfahren nach Anspruch 2, dadurch gekennzeichnet, daß als zum Anionenaustausch befähigte Gruppen tertiäre Aminogruppen und/oder quaternäre Ammoniumgruppen vorgesehen sind.

4. Verfahren nach Anspruch 1, dadurch gekennzeichnet, daß Gruppen vorgesehen sind, die zum Kationenaustausch befähigt sind.

5. Verfahren nach Anspruch 4, dadurch gekennzeichnet, daß als zum Kationenaustausch befähigte Gruppen Sulfonsäuregruppen und/oder Carboxylgruppen vorgesehen sind.

6. Verfahren nach Anspruch 1, dadurch gekennzeichnet, daß als zum Ionenaustausch befähigte Gruppen tertiäre Aminogruppen und/oder quaternäre Ammoniumgruppen und/oder Sulfonsäuregruppen vorgesehen sind.

7. Verwendung von Cellulose, welche mit Gruppen funktionalisiert ist, die zum Ionenaustausch befähigt sind, zum Reinigen einer wäßrigen Lösung eines tertiären Aminoxides, insbesondere eines Fällbades, welches als tertiäres Aminoxid N-Methylmorpholin-N-oxid enthält.

## Claims

1. Process for producing cellulosic moulded bodies, which process comprises the following steps:
(A) dissolving cellulose in an aqueous solution of a tertiary amine oxide, in particular N-methylmorpholine N-oxide, in order to form a mouldable cellulose solution,
(B) moulding the cellulose solution and passing the moulded cellulose solution into an aqueous precipitation bath, in which the cellulose is precipitated, as a result of which a moulded body and a spent precipitation bath are formed,
(C) purifying the spent precipitation bath by bringing the precipitation bath into contact with an ion exchanger, in which process a purified aqueous amine oxide solution is formed which is used again in step (A) for dissolving cellulose, if necessary after concentrating,
characterized
in that an ion exchanger is used whose support is cellulose to which groups capable of ion exchange are attached.

2. Process according to Claim 1, characterized in that groups are provided which are capable of anion exchange.

3. Process according to Claim 2, characterized in that tertiary amino groups and/or quaternary ammonium groups are provided as groups capable of anion exchange.

4. Process according to Claim 1, characterized in that groups are provided which are capable of cation exchange.

5. Process according to Claim 4, characterized in that sulphonic acid groups and/or carboxylic groups are provided as groups capable of cation exchange.

6. Process according to Claim 1, characterized in that tertiary amino groups and/or quaternary ammonium groups and/or sulphonic acid groups are provided as groups capable of ion exchange.

7. Use of cellulose which is functionalized with groups which are capable of ion exchange for purifying an aqueous solution of a tertiary amine oxide, in particular a precipitation bath which contains N-methylmorpholine N-oxide as tertiary amine oxide.

## Revendications

1. Procédé de fabrication de corps moulés cellulosiques, lequel procédé présente les étapes suivantes :
(A) dissolution de cellulose dans une solution aqueuse d'un oxyde d'amine tertiaire, notamment de N-oxyde de N-méthylmorpholine, pour constituer une solution de cellulose formable,
(B) formage de la solution de cellulose et introduction de la solution de cellulose formée dans un bain de précipitation aqueux où la cellulose est précipitée, un corps moulé et un bain de précipitation usé étant ainsi formés,
(C) purification du bain de précipitation usé par mise en contact du bain de précipitation avec un échangeur d'ions, une solution aqueuse purifiée d'oxyde d'amine étant ainsi formée, laquelle, le cas échéant après concentration, est réutilisée à l'étape (A) pour la dissolution de cellulose,
caractérisé en ce qu'est utilisé un échangeur d'ions dont le support est de la cellulose à laquelle sont fixés des groupes capables d'échange d'ions.

2. Procédé selon la revendication 1, caractérisé en ce que sont prévus des groupes capables d'échange d'anions.

3. Procédé selon la revendication 2, caractérisé en ce que sont prévus comme groupes capables d'échange d'anions des groupes amine tertiaire et/ou des groupes ammonium quaternaire.

4. Procédé selon la revendication 1, caractérisé en ce que sont prévus des groupes capables d'échange de cations.

5. Procédé selon la revendication 4, caractérisé en ce que sont prévus comme groupes capables d'échange de cations des groupes acide sulfonique et/ou des groupes carboxyle.

6. Procédé selon la revendication 1, caractérisé en ce que sont prévus comme groupes capables d'échange d'ions des groupes amine tertiaire et/ou des groupes ammonium quaternaire et/ou des groupes acide sulfonique.

7. Utilisation de cellulose fonctionnalisée par des groupes capables d'échange d'ions pour la purification d'une solution aqueuse d'un oxyde d'amine tertiaire, notamment d'un bain de précipitation, contenant comme oxyde d'amine tertiaire du N-oxyde de N-méthylmorpholine.
